# EUROPEAN PATENT APPLICATION

(11) **EP 3 871 670 A1**
(43) Date of publication of application: **01.09.2021**
(21) Application number: 19873233.1
(22) Date of filing: 17.10.2019
(51) Int. Cl.: A61K 31/194, A61P 3/10, A61P 13/12, A61P 29/00, A61P 43/00, A61K 9/20

(54) **INHIBITOR OF RENAL FIBROSIS IN DIABETIC NEPHROPATHY**

(30) Priority: 17.10.2018 JP 2018195826
(71) Applicant: Kyoto Prefectural Public University Corporation, Kyoto-shi, Kyoto 602-8566 (JP); Nippon Chemiphar Co., Ltd., Tokyo 101-0032 (JP)
(72) Inventor: FUKUI, Michiaki, Kyoto-shi, Kyoto 602-8566 (JP); HASHIMOTO, Yoshitaka, Kyoto-shi, Kyoto 602-8566 (JP); NAKAI, Toshiki, Tokyo 101-0032 (JP); UEYAMA, Shigeki, Tokyo 101-0032 (JP); YAMAUCHI, Noboru, Tokyo 101-0032 (JP)
(74) Representative: Ladendorf, Oliver
(86) International application number: PCT/JP2019/040790
(87) International publication number: WO 2020/080451

(57) **Abstract**

The present invention provides a composition including citric acid, a pharmaceutically acceptable salt of citric acid, a hydrate of citric acid, a hydrate of the pharmaceutically acceptable salt of citric acid, or a mixture thereof. Administration or ingestion of the previous period composition inhibits renal fibrosis in diabetic nephropathy.

SEQUENCE LISTING

<110>  Nippon Chemiphar Co., Ltd.

<120>  Renal fibrosis inhibitor for diabetic nephropathy

<130>  UTU9

<140>  JP2018-195826
<141>  2018-10-17

<160>  8

<170>  PatentIn version 3.5

<210>  1
<211>  25
<212>  DNA
<213>  Mus musculus

<400>  1
gtgtggagca acatgtggaa ctcta                                          25


<210>  2
<211>  21
<212>  DNA
<213>  Mus musculus

<400>  2
cgctgaatcg aaagccctgt a                                              21


<210>  3
<211>  25
<212>  DNA
<213>  Mus musculus

<400>  3
aatctgttcc cattagcagg tgaag                                          25


<210>  4
<211>  23
<212>  DNA
<213>  Mus musculus

<400>  4
tgccatgtac cagaatcaaa cca                                            23


<210>  5
<211>  20
<212>  DNA
<213>  Mus musculus

<400>  5
agcagcaggt gtcccaaaga                                                20


<210>  6
<211>  22
<212>  DNA

<213>  Mus musculus

<400>  6
gtgctgaaga ccttagggca ga                                                22


<210>  7
<211>  24
<212>  DNA
<213>  Mus musculus

<400>  7
gcagacgtgg taatgtccaa acag                                              24


<210>  8
<211>  25
<212>  DNA
<213>  Mus musculus

<400>  8
acatcttggc agtatttgtg gtgaa                                             25

## Description

### Technical Field

The present invention relates to the use of citric acid, a pharmaceutically acceptable salt of citric acid, a hydrate of citric acid, a hydrate of the pharmaceutically acceptable salt of citric acid, or a mixture thereof, for example, for inhibiting renal fibrosis in diabetic nephropathy.

### Background Art

Chronic kidney disease (CKD) is a pathology in which findings suggestive of renal damage and a decrease in GFR continue for 3 months or more, and is a concept used irrespective of the primary disease. Diabetic nephropathy, chronic glomerulonephritis, and hypertensive nephrosclerosis are known as the three major causative diseases of CKD that progress to end-stage renal failure. Because renal fibrosis is commonly observed in the process of progression of these diseases to end-stage renal failure, and the degree of renal fibrosis correlates significantly with the prognosis of renal function (Non Patent Literature 1), agents that inhibit renal fibrosis are expected to be promising as inhibitors of progression of CKD.

In recent years, the mechanism of renal fibrosis has been elucidated, and it has been clarified that the cytokine TGF-β1 plays a central role as an inducer of renal fibrosis (Non Patent Literature 2). In fact, based on the inhibitory effect on renal fibrosis in animal experiments, a clinical trial for pirfenidone, which exhibits an inhibitory effect on TGF-β1 action, has been performed on patients with diabetic nephropathy, and as a result, it has been shown that renal function (eGFR) is significantly improved by 1-year medication (Non Patent Literature 3).

It has also been described that the urinary concentration of MCP1, which is a chemokine that induces the migration of monocytes and basophils, increases in renal diseases such as lupus nephritis and IgA nephropathy, in which inflammation is involved in the onset and progression (Non Patent Literature 4), and it has also been described that administration of an antagonist of CCR2, a receptor for MCP1, inhibits renal fibrosis and macrophage infiltration in a mouse model (Non Patent Literature 5).

Further, it has been described that administration of an inhibitor of HIF1α, which is a hypoxia-inducible factor, inhibits renal fibrosis in mice (Non Patent Literature 6).

### Citation List

### Non Patent Literature

Non Patent Literature 1: Nephrol Dial Transplant 16: 765-770, 2001
Non Patent Literature 2: Clin Sci 124: 243-254, 2013
Non Patent Literature 3: J Am Soc Nephrol 24: 1599-1616, 2013
Non Patent Literature 4: Curr Med Chem Anti-Inflammatory & Anti-Allergy, 2: 175-190, 2003
Non Patent Literature 5: J Am Soc Nephrol 15: 940-948, 2004
Non Patent Literature 6: Am J Physiol Renal Physiol 295: F1023-1029, 2008

### Summary of Invention

### Technical Problem

An object of the present invention is, for example, to provide an agent for inhibiting renal fibrosis in diabetic nephropathy.

### Solution to Problem

As a result of intensive studies to solve the above-mentioned problems, the present inventors have found that citric acid, a pharmaceutically acceptable salt of citric acid, a hydrate of citric acid, a hydrate of the pharmaceutically acceptable salt of citric acid, or a mixture thereof is useful, for example, for inhibiting renal fibrosis in diabetic nephropathy, thereby completing the present invention.

In one aspect, the present invention provides a composition for treatment or prevention of renal fibrosis in diabetic nephropathy, including: citric acid, a pharmaceutically acceptable salt of citric acid, a hydrate of citric acid, a hydrate of the pharmaceutically acceptable salt of citric acid, or a mixture thereof.

In one aspect, the present invention provides a composition for inhibiting the production of at least one protein selected from the group consisting of TGF-β, HIF1, and MCP1 in the kidney of patients with diabetic nephropathy, including citric acid, a pharmaceutically acceptable salt of citric acid, a hydrate of citric acid, a hydrate of the pharmaceutically acceptable salt of citric acid, or a mixture thereof.

### Advantageous Effects of Invention

The composition provided by the present invention is useful, for example, for inhibiting renal fibrosis in diabetic nephropathy. The composition provided by the present invention has few side effects and is safe.

### Description of Embodiments

The composition provided by the present invention can include citric acid or a hydrate thereof, a pharmaceutically acceptable salt of citric acid or a hydrate thereof, or a mixture thereof as an active ingredient. Examples of the pharmaceutically acceptable salt of citric acid include alkali metal salts of citric acid. Examples of alkali metal salts of citric acid include potassium citrate and sodium citrate, which can be each hydrates such as stable potassium citrate monohydrate (C₆H₅K₃O₇·H₂O) and sodium citrate dihydrate (C₆H₅Na₃O₇·2H₂O).

Examples of the preferable active ingredient include anhydrous citric acid, sodium citrate, potassium citrate, a hydrate thereof, and a mixture thereof, and the active ingredient can be, for example, a mixture of potassium citrate monohydrate (C₆H₅K₃O₇·H₂O) and sodium citrate dihydrate (C₆H₅Na₃O₇·2H₂O); or a mixture of potassium citrate monohydrate (C₆H₅K₃O₇·H₂O), sodium citrate dihydrate (C₆H₅Na₃O₇·2H₂O), and anhydrous citric acid. The mixing ratio of potassium citrate monohydrate (C₆H₅K₃O₇·H₂O) to sodium citrate dihydrate (C₆H₅Na₃O₇·2H₂O) can be appropriately set by those skilled in the art, and for example, the molar ratio of potassium citrate monohydrate to sodium citrate dihydrate can be 1 : 0.01 to 100. The mixing ratio can be about 1 : 1 in molar ratio.

The mixing ratio of potassium citrate monohydrate (C₆H₅K₃O₇·H₂O) : sodium citrate dihydrate (C₆H₅Na₃O₇·2H₂O) : anhydrous citric acid in a mixture of potassium citrate monohydrate (C₆H₅K₃O₇·H₂O), sodium citrate dihydrate (C₆H₅Na₃O₇·2H₂O), and anhydrous citric acid can be appropriately set by those skilled in the art, and for example, the molar ratio of potassium citrate monohydrate : sodium citrate dihydrate : anhydrous citric acid can be 1 : 0.01 to 100 : 0.01 to 100. The mixing ratio can be about 2 : 2 : 1 in molar ratio.

Other examples of the preferable active ingredient include sodium citrate and a hydrate thereof, and the active ingredient can be, for example, a sodium citrate dihydrate (C₆H₅Na₃O₇·2H₂O).

In addition, other examples of the preferable active ingredient include potassium citrate or a hydrate thereof, and can be, for example, potassium citrate monohydrate (C₆H₅K₃O₇·H₂O).

As an active ingredient, sodium hydrogencarbonate (baking soda) can be used instead of citric acid or a hydrate thereof, a pharmaceutically acceptable salt of citric acid or a hydrate thereof, or a mixture thereof.

In one embodiment, the active ingredient of the composition provided by the present invention is citric acid or a hydrate thereof, a pharmaceutically acceptable salt of citric acid or a hydrate thereof, or a mixture thereof, and the pharmaceutically acceptable salt of citric acid is a salt other than ferric citrate.

In one embodiment, the active ingredient of the composition provided by the present invention can include citric acid or a hydrate thereof, and a mixture of sodium citrate or a hydrate thereof and potassium citrate or a hydrate thereof, and can be composed only of citric acid or a hydrate thereof, and a mixture of sodium citrate or a hydrate thereof and potassium citrate or a hydrate thereof.

In one embodiment, the active ingredient of the composition provided by the present invention can include a mixture of sodium citrate or a hydrate thereof and potassium citrate or a hydrate thereof, and can be composed only of a mixture of sodium citrate or a hydrate thereof and potassium citrate or a hydrate thereof.

In the present specification, when referring to the weight of citric acid or a hydrate thereof, a pharmaceutically acceptable salt of citric acid or a hydrate thereof, or a mixture thereof (for example, a potassium citrate monohydrate (C₆H₅K₃O₇·H₂O) and sodium citrate dihydrate (C₆H₅Na₃O₇·2H₂)), the weight can be dry weight.

The composition provided by the present invention can be a medicine, and is useful for inhibiting renal fibrosis in diabetic nephropathy, inhibiting inflammation associated with renal fibrosis in the kidney of a patient with diabetic nephropathy, or inhibiting the production of TGF-β, HIF1 and/or MCP1 in the kidney of a patient with diabetic nephropathy.

In the present specification, examples of diabetes mellitus include, but are not limited to, type 1 diabetes mellitus and type 2 diabetes mellitus.

The subject of administration of the composition (pharmaceutical composition), the medicine provided by the present invention, can be appropriately selected by a doctor.

In one embodiment, the subject of administration of the pharmaceutical composition provided by the present invention can be a person suffering from diabetic nephropathy and a person at risk of developing diabetic nephropathy (for example, a person who has not developed diabetic nephropathy, but is suffering from diabetes mellitus).

In one embodiment, administration of the pharmaceutical composition provided by the present invention to a patient with diabetic nephropathy can inhibit the exacerbation of renal fibrosis compared to administration of a placebo.

In one embodiment, administration of the pharmaceutical composition provided by the present invention to a patient with diabetic nephropathy can inhibit the exacerbation of inflammation associated with renal fibrosis in the kidney compared to administration of a placebo.

In one embodiment, administration of the pharmaceutical composition provided by the present invention to a patient with diabetic nephropathy can inhibit the exacerbation of renal fibrosis compared to before administration.

In one embodiment, administration of the pharmaceutical composition provided by the present invention to a patient with diabetic nephropathy can inhibit the exacerbation of inflammation associated with renal fibrosis in the kidney compared to before administration.

The pharmaceutical composition provided by the present invention inhibits expression of at least one protein selected from the group consisting of TGF-β, HIF1, and MCP1 in a kidney. For example, the pharmaceutical composition provided by the present invention inhibits expression of protein TGF-β, HIF1, or MCP1 in a kidney. In one embodiment, administration of the pharmaceutical composition provided by the present invention to a patient with diabetic nephropathy inhibits expression of protein TGF-β in a kidney compared to administration of a placebo.

In one embodiment, administration of the pharmaceutical composition provided by the present invention to a patient with diabetic nephropathy inhibits expression of protein HIF1 in a kidney compared to administration of a placebo.

In one embodiment, administration of the pharmaceutical composition provided by the present invention to a patient with diabetic nephropathy inhibits expression of protein MCP1 in a kidney compared to administration of a placebo.

In one embodiment, administration of the pharmaceutical composition provided by the present invention to a patient with diabetic nephropathy inhibits expression of protein TGF-β in a kidney compared to before administration.

In one embodiment, administration of the pharmaceutical composition provided by the present invention to a patient with diabetic nephropathy inhibits expression of protein HIF1 in a kidney compared to before administration.

In one embodiment, administration of the pharmaceutical composition provided by the present invention to a patient with diabetic nephropathy inhibits expression of protein MCP1 in a kidney compared to before administration.

Expression of proteins can be evaluated by methods known to those skilled in the art, and can be evaluated by, for example, methods in which quantitative PCR (for example, quantitative RT-PCR), ELISA, Western blotting, or LC-MS/MS is used.

Fibrosis in a kidney can be evaluated pathologically, for example, using renal tissue obtained by a renal biopsy.

In one embodiment, inhibition of expression of at least one protein selected from the group consisting of TGF-β, HIF1, and MCP1 in renal tissue can be understood as inhibition of the progression of renal fibrosis.

In one embodiment, inhibition of expression of at least one protein selected from the group consisting of TGF-β and MCP1 in renal tissue can be understood as inhibition of inflammation associated with the progression of renal fibrosis.

In one embodiment, the pharmaceutical composition provided by the present invention is used for uric acid control in a patient with chronic kidney disease, has few side effects, and is safe.

In one embodiment, the pharmaceutical composition provided by the present invention pathology-specifically or partially inhibits the action of TGF-β, HIF1, and MCP1, and is thus safe, and has few side effects.

In one embodiment, the pharmaceutical composition provided by the present invention does not inhibit expression of at least one protein selected from the group consisting of TGF-β, HIF1, and MCP1 by 50% or more or 60% or more compared to administration of a placebo when the pharmaceutical composition provided by the present invention is administered.

In one embodiment, the pharmaceutical composition provided by the present invention does not inhibit expression of proteins TGF-β, HIF1, and MCP1 by 50% or more or 60% or more compared to administration of a placebo when the pharmaceutical composition provided by the present invention is administered.

In one embodiment, the pharmaceutical composition provided by the present invention inhibits expression of at least one protein selected from the group consisting of TGF-β, HIF1, and MCP1 by 10% to 40% compared to administration of a placebo when the pharmaceutical composition provided by the present invention is administered.

In one embodiment, the pharmaceutical composition provided by the present invention inhibits expression of proteins TGF-β, HIF1, and MCP1 by 10% to 40% compared to administration of a placebo when the pharmaceutical composition provided by the present invention is administered.

The pharmaceutical composition provided by the present invention is orally or parenterally administered to humans or other mammals, and examples of parenteral administration include intravenous administration, subcutaneous administration, intramuscular administration, intraarticular administration, transmucosal administration, transdermal administration, nasal administration, rectal administration, intrathecal administration, intraperitoneal administration, and local administration. The dose of the active ingredient (for example, citric acid or a hydrate thereof; potassium citrate or a hydrate thereof; sodium citrate or a hydrate thereof; a mixture of potassium citrate monohydrate and sodium citrate dihydrate; a mixture of citric acid, potassium citrate monohydrate, and sodium citrate dihydrate; or sodium hydrogencarbonate) can be appropriately set by those skilled in the art.

The pharmaceutical composition provided by the present invention can be prepared using the active ingredient (for example, citric acid or a hydrate thereof; potassium citrate or a hydrate thereof; sodium citrate or a hydrate thereof; a mixture of potassium citrate monohydrate and sodium citrate dihydrate; a mixture of citric acid, potassium citrate monohydrate, and sodium citrate dihydrate; or sodium hydrogencarbonate) as it is or by mixing the active ingredient with a pharmaceutically acceptable carrier such as an excipient (for example, lactose, D-mannitol, crystalline cellulose, and glucose), a binder (for example, hydroxypropylcellulose (HPC), gelatin, and polyvinylpyrrolidone (PVP)), a lubricant (for example, magnesium stearate and talc), a disintegrant (for example, starch and carboxymethylcellulose calcium (CMC-Ca)), a diluent (for example, water for injection and saline), and other additives (for example, a pH regulator, a surfactant, a solubilizer, a preservative, an emulsifier, a tonicity adjusting agent, and a stabilizing agent) if necessary, and can be a preparation such as a tablet, a capsule, a suspension, an injection, and a suppository. For example, when the pharmaceutical composition is a tablet, the pharmaceutical composition can be formulated by mixing the active ingredient (for example, citric acid or a hydrate thereof; potassium citrate or a hydrate thereof; sodium citrate or a hydrate thereof; a mixture of potassium citrate monohydrate and sodium citrate dihydrate; a mixture of citric acid, potassium citrate monohydrate, and sodium citrate dihydrate; or sodium hydrogencarbonate) with an excipient (for example, lactose, D-mannitol, crystalline cellulose, and glucose), a disintegrant (for example, starch and carboxymethylcellulose calcium (CMC-Ca)), a binder (for example, hydroxypropylcellulose (HPC), gelatin, and polyvinylpyrrolidone (PVP)), a lubricant (for example, magnesium stearate and talc) or the like. The tablet can be an uncoated tablet or a film-coated tablet.

In one embodiment, the pharmaceutical composition provided by the present invention is a tablet. The tablet provided by the present invention include, in addition to the active ingredient (for example, citric acid or a hydrate thereof; potassium citrate or a hydrate thereof; sodium citrate or a hydrate thereof; a mixture of potassium citrate monohydrate and sodium citrate dihydrate; a mixture of citric acid, potassium citrate monohydrate, and sodium citrate dihydrate; or sodium hydrogencarbonate), an additive that is conventional in the pharmaceutical field and pharmaceutically acceptable. Examples of such an additive include an excipient, a binder, a disintegrant, a fluidizer, a corrigent, a lubricant, a pH regulator, a surfactant, a stabilizing agent, and a flavor.

In one embodiment, when the active ingredient of the pharmaceutical composition (for example, a tablet) provided by the present invention is an alkali metal salt of citric acid (for example, potassium citrate or a hydrate thereof (for example, potassium citrate monohydrate); sodium citrate or a hydrate thereof (for example, sodium citrate dihydrate); a mixture of potassium citrate or a hydrate thereof and sodium citrate or a hydrate thereof; or a mixture of potassium citrate monohydrate and sodium citrate dihydrate), the pharmaceutical composition provided by the present invention (for example, a tablet) can include anhydrous citric acid as a stabilizing agent.

The content of the active ingredient (for example, citric acid or a hydrate thereof; potassium citrate or a hydrate thereof; sodium citrate or a hydrate thereof; a mixture of potassium citrate monohydrate and sodium citrate dihydrate; a mixture of citric acid, potassium citrate monohydrate, and sodium citrate dihydrate; or sodium hydrogencarbonate) in a tablet provided by the present invention can be 10 to 95% by weight, preferably 30 to 90% by weight, and more preferably 60 to 90% by weight based on the weight of the tablet.

The pharmaceutical composition provided by the present invention can be produced by a method known in the pharmaceutical field. For example, in the case of a tablet, the method for production can include a mixing step of mixing an active ingredient (for example, potassium citrate or a hydrate thereof; sodium citrate or a hydrate thereof; mixture of potassium citrate monohydrate and sodium citrate dihydrate; or sodium hydrogencarbonate) with an additive agent; a granulating step; a tableting step; and/or a coating step.

The mixing step can include a step of mixing an active ingredient with an additive agent such as an excipient, a stabilizing agent, a disintegrant, and/or a binder. The method for production can further include a step of mixing a mixture including an active ingredient and an additive with a lubricant, a corrigent, and/or a flavor before the tableting step. Mixing can be performed using a V-type mixer, a W-type mixer, a container mixer, a tumbler mixer, a stirring mixer or the like.

The granulating step can be performed by a granulating method known in the pharmaceutical field. Examples of the granulating method include a dry granulating method, a wet granulating method, and a fluidized bed granulating method.

In one embodiment, the mixture obtained in the mixing step and the granulated product obtained in the granulating step are appropriately grinded and/or sieved to obtain a mixture or granulated product having a desired particle size. Grinding can be performed by, for example, a grinder known in the pharmaceutical field such as a ball mill, a jet mill, and a hammer mill. Sieving can be performed using a sieve of 16 mesh (opening: 1000 µm) to 32 mesh (opening: 500 µm) and the like.

The tableting step can be performed by a tableting method known in the pharmaceutical field. Examples of the tableting method include direct tableting method, dry tableting method, wet tableting method, and external lubrication tableting method. The mixture or granulated product obtained in the above-mentioned step can be tableted using, for example, a tableting machine known in the pharmaceutical field such as a single punch tableting machine and a rotary tableting machine. When a single punch tableting machine, a rotary tableting machine or the like is used, a tableting pressure of 1 kN to 30 kN can be employed.

The coating step can be performed by a method known in the pharmaceutical field. For example, the coating step can be performed by spray-coating the outside of an uncoated tablet with a coating liquid containing a coating base; and a plasticizer, a colorant, a brightener and the like as appropriate.

In one embodiment, the hardness of the resulting tablet can be 10 to 200 N, preferably 30 to 150 N.

The amount of the active ingredient (for example, potassium citrate or a hydrate thereof; sodium citrate or a hydrate thereof; a mixture of potassium citrate monohydrate and sodium citrate dihydrate; or sodium hydrogencarbonate) in the pharmaceutical composition provided by the present invention can be appropriately set.

In one embodiment, the pharmaceutical composition provided by the present invention is a tablet, and one tablet can contain 10 mg to 1 g, preferably contain 100 mg to 600 mg, and more preferably contain 400 mg to 550 mg of anhydrous citric acid, potassium citrate monohydrate or sodium citrate dihydrate as an active ingredient.

In one embodiment, the pharmaceutical composition provided by the present invention is a tablet, and one tablet can contain 10 mg to 300 mg each, 20 mg to 600 mg in total, of anhydrous citric acid, potassium citrate monohydrate, and sodium citrate dihydrate, preferably contain 70 to 250 mg each, 400 to 600 mg in total, of anhydrous citric acid, potassium citrate monohydrate, and sodium citrate dihydrate, and more preferably contain 70 to 240 mg each, 450 to 550 mg in total, of anhydrous citric acid, potassium citrate monohydrate, and sodium citrate dihydrate.

In one embodiment, the pharmaceutical composition provided by the present invention is a tablet, and one tablet can contain 10 mg to 1 g of, and preferably contain 100 mg to 500 mg of sodium hydrogencarbonate as an active ingredient.

In one embodiment, the pharmaceutical composition provided by the present invention is a tablet, and can contain 231.5 mg of potassium citrate monohydrate and 195.0 mg of sodium citrate dihydrate as active ingredients, and anhydrous citric acid, crystalline cellulose, partly pregelatinized starch, hydroxypropyl cellulose, magnesium stearate, hypromerose, macrogol 6000, titanium oxide, and carnauba wax as additives.

The composition provided by the present invention can be used as a food. The food can be ingested by humans or other mammals (for example, healthy humans, healthy mammals) as long as treatment or prevention of a disease is not intended.

By ingestion of the food provided by the present invention, an effect of, for example, maintaining health of a kidney can be obtained.

The food provided by the present invention can be a food for specified health use, a food with nutrient function claims, or a food with functional claims.

The form of the food is not particularly limited as long as the food can be ingested orally, and the form can be a supplement or a general food. Examples of the general food include beverages (for example, beverages containing fruit extract or vegetable extract such as juice, tea beverages, sports beverages, flavored waters, and diet beverages), candy, jelly, gummi candy, and gum. The food provided by the present invention can be appropriately produced by those skilled in the art according to the type of food, and can be produced, for example, by incorporating citric acid or a hydrate thereof, a pharmaceutically acceptable salt of citric acid or a hydrate thereof, or a mixture thereof in a food material. Those skilled in the art can also appropriately set the content of citric acid or a hydrate thereof, a pharmaceutically acceptable salt of citric acid or a hydrate thereof, or a mixture thereof contained in the food. For example, when the food contains citric acid, potassium citrate and/or sodium citrate hydrate, the food can contain citric acid, potassium citrate and/or sodium citrate hydrate so that 1 to 6 g in total of anhydrous citric acid, potassium citrate, or sodium citrate hydrate will be ingested per day, and preferably, 1 to 3 g in total of anhydrous citric acid, potassium citrate, or sodium citrate hydrate will be ingested per day. When the food provided by the present invention is a tablet, the tablet can be produced according to the above-mentioned method for producing a tablet in the pharmaceutical field so that, for example, 70 to 90% by weight of citric acid or a hydrate thereof, a pharmaceutically acceptable salt of citric acid or a hydrate thereof, or a mixture thereof (for example, citric acid, potassium citrate and sodium citrate hydrate) will be contained per tablet having a weigh of 300 mg to 600 mg.

In one embodiment, the present invention provides use of citric acid or a hydrate thereof, a pharmaceutically acceptable salt of citric acid or a hydrate thereof, or a mixture thereof for the production of the pharmaceutical composition or food provided by the present invention.

In one embodiment, the present invention provides use of citric acid or a hydrate thereof, a pharmaceutically acceptable salt of citric acid or a hydrate thereof, or a mixture thereof for the production of a pharmaceutical composition for inhibiting renal fibrosis in diabetic nephropathy.

In one embodiment, the present invention provides use of citric acid or a hydrate thereof, a pharmaceutically acceptable salt of citric acid or a hydrate thereof, or a mixture thereof for the production of a food for maintaining renal health.

In one embodiment, the present invention provides use of sodium hydrogencarbonate for the production of the pharmaceutical composition or food provided by the present invention.

In one embodiment, the present invention provides use of sodium hydrogencarbonate for the production of a pharmaceutical composition for inhibiting renal fibrosis in diabetic nephropathy.

In one embodiment, the present invention provides use of sodium hydrogencarbonate for the production of a food for maintaining renal health.

Examples of the embodiments provided by the present invention include (1-1) to (1-8), (2-1) to (2-8), and (3-1) to (3-8) below.
(1-1)
   A pharmaceutical composition, including: citric acid, a pharmaceutically acceptable salt of citric acid, a hydrate of citric acid, a hydrate of the pharmaceutically acceptable salt of citric acid, or a mixture thereof as an active ingredient for use in inhibition of renal fibrosis in diabetic nephropathy;
(1-2)
   The pharmaceutical composition according to (1-1), which inhibits inflammation in a kidney;
(1-3)
   The pharmaceutical composition according to (1-1) or (1-2), which inhibits expression of at least one protein selected from the group consisting of TGF-β, HIF1, and MCP1 in a kidney;
(1-4)
   The pharmaceutical composition according to any one of (1-1) to (1-3), which is a tablet;
(1-5)
   The pharmaceutical composition according to any one of (1-1) to (1-4), wherein the citric acid, the pharmaceutically acceptable salt of citric acid, the hydrate of citric acid, the hydrate of the pharmaceutically acceptable salt of citric acid, or the mixture thereof is anhydrous citric acid, a pharmaceutically acceptable salt of citric acid, a hydrate of thereof, or a mixture thereof;
(1-6)
   The pharmaceutical composition according to any one of (1-1) to (1-5), wherein the citric acid, the pharmaceutically acceptable salt of citric acid, the hydrate of citric acid, the hydrate of the pharmaceutically acceptable salt of citric acid, or the mixture thereof is a mixture of sodium citrate or a hydrate thereof and potassium citrate or a hydrate thereof.
(1-7)
   The pharmaceutical composition according to any one of (1-1) to (1-6), which is administered to a patient suffering from type 1 diabetes mellitus.
(1-8)
   A food composition, including: citric acid, a pharmaceutically acceptable salt of citric acid, a hydrate of citric acid, a hydrate of the pharmaceutically acceptable salt of citric acid, or a mixture thereof, for use in maintaining renal health.
(2-1)
   Use of citric acid, a pharmaceutically acceptable salt of citric acid, a hydrate of citric acid, a hydrate of the pharmaceutically acceptable salt of citric acid, or a mixture thereof as an active ingredient in the production of a pharmaceutical composition for inhibiting renal fibrosis in diabetic nephropathy;
(2-2)
   The use according to (2-1), wherein the pharmaceutical composition inhibits inflammation in a kidney;
(2-3)
   The use according to (2-1) or (2-2), wherein the pharmaceutical composition further inhibits expression of at least one protein selected from the group consisting of TGF-β, HIF1, and MCP1 in a kidney;
(2-4)
   The use according to any one of (2-1) to (2-3), wherein the pharmaceutical composition is a tablet;
(2-5)
   The use according to any one of (2-1) to (2-4), wherein the citric acid, the pharmaceutically acceptable salt of citric acid, the hydrate of citric acid, the hydrate of the pharmaceutically acceptable salt of citric acid, or the mixture thereof is anhydrous citric acid, a pharmaceutically acceptable salt of citric acid, a hydrate thereof, or a mixture thereof;
(2-6)
   The use according to any one of (2-1) to (2-5), wherein the citric acid, the pharmaceutically acceptable salt of citric acid, the hydrate of citric acid, the hydrate of the pharmaceutically acceptable salt of citric acid, or the mixture thereof is a mixture of sodium citrate or a hydrate thereof and potassium citrate or a hydrate thereof.
(2-7)
   The use according to any one of (2-1) to (2-6), wherein the pharmaceutical composition is administered to a patient suffering from type 1 diabetes mellitus.
(2-8)
   Use of citric acid, a pharmaceutically acceptable salt of citric acid, a hydrate of citric acid, a hydrate of the pharmaceutically acceptable salt of citric acid, or a mixture thereof in production of a food composition for maintaining renal health in a human.
(3-1)
   A method for inhibiting renal fibrosis in diabetic nephropathy in a mammalian subject (for example, a human), including the step of: administering an effective amount of a food composition including citric acid, a pharmaceutically acceptable salt of citric acid, a hydrate of citric acid, a hydrate of the pharmaceutically acceptable salt of citric acid, or a mixture thereof to a subject in need of inhibiting renal fibrosis in diabetic nephropathy;
(3-2)
   The method according to (3-1), which further inhibits inflammation in a kidney;
(3-3)
   The method according to (3-1) or (3-2), which further inhibits expression of at least one protein selected from the group consisting of TGF-β, HIF1, and MCP1 in a kidney;
(3-4)
   The method according to any one of (3-1) to (3-3), wherein the pharmaceutical composition is a tablet;
(3-5)
   The method according to any one of (3-1) to (3-4), wherein the citric acid, the pharmaceutically acceptable salt of citric acid, the hydrate of citric acid, the hydrate of the pharmaceutically acceptable salt of citric acid, or the mixture thereof is anhydrous citric acid, a pharmaceutically acceptable salt of citric acid, a hydrate thereof, or a mixture thereof;
(3-6)
   The method according to any one of (3-1) to (3-5), wherein the citric acid, the pharmaceutically acceptable salt of citric acid, the hydrate of citric acid, the hydrate of the pharmaceutically acceptable salt of citric acid, or the mixture thereof is a mixture of sodium citrate or a hydrate thereof and potassium citrate or a hydrate thereof;
(3-7)
   The method according to any one of (3-1) to (3-6), wherein the pharmaceutical composition is administered to a patient suffering from type 1 diabetes mellitus;
(3-8)
   A method for maintaining renal health in a mammalian subject (for example, a human), including the step of: making a subject in need of maintaining renal health ingest an effective amount of a pharmaceutical composition including citric acid, a pharmaceutically acceptable salt of citric acid, a hydrate of citric acid, a hydrate of the pharmaceutically acceptable salt of citric acid, or a mixture thereof.

Hereinafter, though the present invention will be further described with reference to Examples, the present invention is not limited thereto.

### Examples

C57BL/6N male mice were randomly divided into 3 groups (Control group, Citrate (-) group, Citrate (+) group) consisting of 3 mice. After fasting the animals for 24 hours, citrate buffer (10 mM, pH 4.5) was intraperitoneally administered in a single dose to the Control group, and streptozotocin (STZ) dissolved in citrate buffer (10 mM, pH 4.5) (200 mg/kg; Sigma-Aldrich, USA) was intraperitoneally administered in a single dose to the Citrate (-) group and the Citrate (+) group. On the 20th day after STZ administration, the blood glucose level in the blood sample obtained from the tail was measured using a blood glucose meter to confirm that the animals have developed diabetes mellitus.

An aqueous solution containing citrate (an aqueous solution containing 463 mg of potassium citrate monohydrate and 390 mg of sodium citrate dihydrate in 100 mL) was prepared, and the mice in the Citrate (+) group were freely fed the aqueous solution for a week. Tap water was administered as drinking water to the mice in the Control group and Citrate (-) group.

The urine pH was measured one week after the administration as drinking water. While the urine pH of the mice in the Citrate (-) group was 5.296, the urine pH of the mice in the Citrate (+) group was 5.568, and was confirmed to be alkalinized.

One week after the administration as drinking water, kidneys were removed from the mice to prepare cDNA. Using this as a template, quantitative PCR was performed using the primers of TGF-β, HIF1, MCP1, and Sirt1 (Table 2) (amplification products were 2191 bp, 4775 bp, 175 bp and 3793 bp, respectively), and the expression levels of TGF-β, HIF1, MCP1, and Sirt1 in the kidney were determined as relative amounts to the internal standard.

**[Table 1]**

| Effects of Citrate un the Pathological Markers of Kidney in STZ-induced Diabates Mice | | | | |
|---|---|---|---|---|
| Parameters | Control (Normal) | STZ | | 1-way ANOVA |
| | | Citrate(-) | Citrate(+) | |
| TGF-β | 1.067 ± 0.208 | 1.367 ± 0.551 | 0.867 ± 0.208 | 0.3040 |
| HIF1 | 1.067 ± 0.058 | 1.033± 0.153 | 0.767 ± 0.1.53^{*b,c*,*i*} | 0.0555 |
| MCP1 | 0.500 1. 0.100 | 1.400 ±0.100 | 1.200 ± 0.440*^{d}* | 0.0136 |
| Sirt1 | 1.167±0.118 | 1.100 ± 0.200 | 1.167 ± 0.058 | 0.7973 |

| | | | | |
|---|---|---|---|---|
| Mean +/- SD, n=3 ^{a}p>0.9999 vs Control and ^{b}p=0.0983 vs Citrate(·), ^{c}p=0.0139 vs Control and ^{d}p=0.6458 vs Citrate (·) by Tukey test ^{e}p=0.335 vs Control and ^{r}p=0.0993 vs Citrate(·) by Studenrt test | | | | |

**[Table 2]**

| gene | sequence |
|---|---|
| TGF-β | Forward Primer 5'-GTGTGGAGCAACATGTGGAACTCTA-3' |
| | Reverse Primer 5'-CGCTGAATCGAAAGCCCTGTA-3' |
| | GenBank accession no. NM011577 |
| HIF1. | Forward Primer: 5'-AATCTGTGTTCCCATTAGCAGGTGAAG-3' |
| | Reverse Primer: 5'-TGCCATGTACCAGAATCAAACCA-3' |
| | GenBank secession no. NM010431 |
| MCP1 | Forward Primer: 5'-AGCAGCAGGTGTCCCAAAGA-3' |
| | Reverse Primer: 5'-GTGCTGAAGACCTTAGGGCAGA-3' |
| | GenBank accession no. NM011333 |
| Sirt1 | Forward Primer: 5'-GCAGACGTGGTAATGTCCAAAGAG-3' |
| | Reverse Primer 5'-AGATCTTGGCAGTATTTGTGGTGAA-3' |
| | GenBank accession no. NM001159589 |

As a result, the STZ-induced increase in TGF-β and MCP1 expression levels was inhibited, and the HIF1 expression level decreased in the diabetic mice to which the aqueous solution containing citrate was administered, compared to the group without administration of citrate (Table 1). The expression level of HIF1 in the group with administration of the aqueous solution containing citrate decreased compared to the Control group (Table 1).

The results in Table 1 showed that administration of the aqueous solution containing citrate pathology-specifically or partially inhibits the expression of TGF-β, HIF1, and MCP1. It was also suggested that administration of the aqueous solution containing citrate inhibits renal fibrosis and inflammation in the process of progression in diabetic mice.

### Industrial Applicability

A composition for inhibiting renal fibrosis in diabetic nephropathy and the like are provided by using the present invention.

## Claims

1. A pharmaceutical composition for treatment or prevention of renal fibrosis in diabetic nephropathy, comprising: citric acid, a pharmaceutically acceptable salt of citric acid, a hydrate of citric acid, a hydrate of the pharmaceutically acceptable salt of citric acid, or a mixture thereof, wherein the pharmaceutical composition is a tablet.

2. The pharmaceutical composition according to claim 1, which inhibits inflammation in a kidney.

3. The pharmaceutical composition according to any one of claims 1 or 2, which inhibits expression of at least one protein selected from the group consisting of TGF-β, HIF1, and MCP1 in a kidney.

4. The pharmaceutical composition according to any one of claims 1 to 3, wherein the citric acid, the pharmaceutically acceptable salt of citric acid, the hydrate of citric acid, the hydrate of the pharmaceutically acceptable salt of citric acid, or the mixture thereof is a mixture of anhydrous citric acid, sodium citrate or a hydrate thereof, and potassium citrate or a hydrate thereof.

5. The pharmaceutical composition according to any one of claims 1 to 4, wherein the citric acid, the pharmaceutically acceptable salt of citric acid, the hydrate of citric acid, the hydrate of the pharmaceutically acceptable salt of citric acid, or the mixture thereof is anhydrous citric acid, potassium citrate monohydrate, and sodium citrate dihydrate.

6. The pharmaceutical composition according to any one of claims 1 to 5, which is administered to a patient suffering from type 1 diabetes mellitus.
